# EUROPEAN PATENT SPECIFICATION

(11) **EP 4 545 059 B1**
(45) Date of publication and mention of the grant of the patent: **22.10.2025**
(21) Application number: 23213606.9
(22) Date of filing: 01.12.2023
(51) Int. Cl.: A61H 23/02

(54) **DEVICE FOR MASSAGING FULL FACE MULTIFUNCTIONALLY AND MUSICALLY**
MULTIFUNKTIONELLE UND MUSIKALISCHE MASSAGEVORRICHTUNG FÜR DAS GANZE GESICHT
DISPOSITIF DE MASSAGE DE VISAGE ENTIER MULTIFONCTIONNEL ET MUSICAL

(30) Priority: 23.10.2023 CN 202311375700
(43) Date of publication of application: 30.04.2025
(73) Proprietor: Guangzhou Candear Packing Products Co., Ltd, Guangzhou 510000 (CN)
(72) Inventor: Cai, Guoxiang, Guangzhou, 510000 (CN)
(74) Representative: Diaz Nunez, Joaquin

(56) References cited:
- CN-U- 203 802 777
- US-A1- 2021 038 471
- US-B2- 7 282 037

## Description

### REFERENCE TO PRIOR APPLICATION

This application claims priority to Chinese Patent Application 202311375700.4, filed on October 23, 2023.

### TECHNICAL FIELD

The present invention relates to the technical field of health care equipment, and specifically to a device for massaging a full face multifunctionally and musically.

### BACKGROUND

A massaging apparatus is the upgraded version of a massager, and is a new generation of health equipment developed according to physics, bionics, bioelectricity, Chinese medicine and many years of clinical practice.

Many existing massage apparatuses are commonly pushed by a hand to press a face. A vibration amplitude is small and massage strength is not enough. Such a hand-push massage apparatus is time-consuming and laborious during the massage. There is a need for an electric massage apparatus on a market. However, most of the electric massaging apparatuses as a whole is smooth and streamlined. Although a user feels comfortable when her/his skin comes into contact with the electric massaging apparatus, when the user holds the apparatus by her/his hand, apparatus can not be gripped tightly by the hand because the apparatus is too smooth. In addition, a high-frequency vibration can easily lead to the apparatus off the hand. A hand-push massaging apparatus or the electric massaging apparatus has only the basic effect of the massage. Therefore, a device for massaging a full face multifunctionally and musically is proposed. Patent Document US7282037B2 discloses a device for massaging a full face multifunctionally and musically comprising a device body, wherein the device body is provided with a sound outlet hole at one side end face of a periphery.

The prior art has the following defects or problems:
1. The existing face massaging apparatus has too smooth appearance as a whole. A differentiation process is not made on the details of a grip part. The grip part is off hand under a high-intensity vibration. In addition, the existing massaging apparatus is not fit enough with the contact surface of a human body.
2. The existing face massage apparatus, whether it is the hand-push massage apparatus or the electric massage apparatus, has only a basic massage function which is monofunctional. Therefore, the user has monotonous experience when using the existing face massage apparatus.

### SUMMARY

To overcome the deficiencies of the prior art, an objective of the present invention is to provide a device for massaging a full face multifunctionally and musically, in order to solve the problems raised in the background.

In order to realize the forgoing objective, the present invention provides the following technical solution: a device for massaging a full face multifunctionally and musically includes a device body. The device body is provided with a sound outlet hole at one side end face of the periphery. The device body is provided with an anti-slip pad at one side end face of the bottom end. The device body is provided with an upper cover at one side end face of the upper end. The upper cover is provided with a vibrating massaging apparatus at one side end face of the top. The vibrating massaging apparatus is provided with a touch switch at the end face of the center. The upper cover is provided with a TYPE-C charging port at one side end face. The upper cover is provided with an LED light belt on the end face of the upper side of the periphery.

Further, an operating system of the device for massaging the full face multifunctionally and musically is specified as follows: The operating system includes a massage module, a charging module, and a music module. The massage module includes a control module, a motor module, and a vibrating module. The charging module includes a short-circuit protection module and an over-charging and over-discharging protection module. The music module includes a control module, a light module, a sound-generating module, and a Bluetooth module. An output terminal of the motor module in the massage module is connected to the vibrating module. An output terminal of the short-circuit protection module in the charging protection module is connected to the over-charging and over-discharging protection module. An output terminal of the light module in the music module is connected to the sound-generating module. An output terminal of the control module is connected to the motor module and the Bluetooth module. The motor module provides a driving force for the vibrating module. The vibrating module directly contacts a user as a vibrating element. The short-circuit protection module may provide protection in the event of a short circuit in the device. The over-charging and over-discharging protection module may provide protection when the device is overcharged or anti-power. The light module may be used as the light-emitting element of the device. The sound-generating module may be configured to play music. The Bluetooth module may be connected to a cell phone. The control module may be used as a control element of the music module and the massage module.

As a preferable technical solution of the present invention, the device body and a finger grip part of the upper cover are made of a frosted matte ABS plastic.

As a preferable technical solution of the present invention, the device body, the upper cover, and the vibrating massaging apparatus as a whole adopt a heart-shaped triangular external structure with an upward tip. The vibrating massaging apparatus is connected to the device body via a magnetic suction. The vibrating massaging apparatus is movably connected to the device body.

As a preferable technical solution of the present invention, three groups of sound outlet holes are disclosed and distributed at each face of a triangle of the device body. The sound outlet holes are fixedly mounted and connected to the device body.

As a preferable technical solution of the present invention, the upper cover is movably connected to the upper end of the device body by a magnetic suction card.

As a preferable technical solution of the present invention, the vibrating massaging apparatus is fixedly connected to the inner part of the upper cover.

As a preferable technical solution of the present invention, the anti-slip pad is fixedly connected to the bottom end of the device body by bonding.

Compared with the prior art, the present invention provides the device for massaging the full face multifunctionally and musically with the following beneficial effects:
1. The exterior of a part between the device body and the upper cover of the device for massaging the full face multifunctionally and musically, which is gripped by the hand of the user, is provided with a frosted matte ABS plastic plating layer so that a contact friction between the hand of the user and the device can be increased when the user grips the device, so as to prevent the device from slipping out of the hand due to the high-frequency vibration when the device is used.
2. The device for massaging the full face multifunctionally and musically is provided with an external structure with a heart-shaped notch is arranged, which may be further convenient for the user to grip the device by the hand. A structure with a triangular apex is arranged so that the vibrating massaging apparatus is positioned at the highest point of the whole device. Therefore, it is possible to enable the user to align a massaging structure more accurately to any part of a face when the device is used.
3. Because the device for massaging the full face multifunctionally and musically is provided with high-frequency vibration of 15,000 revolutions per minute, when the vibrating massaging apparatus is in contact with the skin of a human face, every inch of the skin is massaged with a high-speed vibration, which can increase the speed of the skin to absorb a cream. The massage apparatus is electroplated and polished at the periphery and the bottom surface so that the user feels comfortable and considerate when the massage apparatus is in contact with the skin.
4. Because the device for massaging the full face multifunctionally and musically is provided with an LED light belt and three groups of the sound outlet holes, when the device is used, a good environmental atmosphere may be created with a music rhythm and a flashing light. Therefore, the device also has a multifunctional combination of music and lighting in addition to a basic massaging effect.
5. The device for massaging the full face multifunctionally and musically is provided with a magnetic suction card, the massaging apparatus with an automatic charging effect, and the device body so that the user may have the effect of quickly opening or closing the upper cover. The LED light belt is arranged, so that the device has relatively independent light display when the device is charged, used, and switched. Therefore, the state of the device is clear at a glance, which further facilitates the use of the user. With a Hall detection switching principle, when the massaging apparatus is put back to the device body, magnetism is detected by Hall, which triggers a circuit to be powered off. When the massaging apparatus is taken away from the device body for use, magnetic disappearance is detected by Hall, which triggers the circuit to be powered on and automatically play music.

### BRIEF DESCRIPTION OF DRAWINGS

Fig. 1 shows a schematic diagram of a front overall structure of the present invention;
Fig. 2 shows a schematic diagram of a transverse planar structure of the present invention;
Fig. 3 shows a schematic diagram of a forward planar structure of the present invention;
Fig. 4 shows a schematic diagram of a lower oblique side structure of the present invention;
Fig. 5 shows a schematic diagram of an upper oblique side structure of the present invention;
Fig. 6 shows a schematic diagram of a top planar structure of the present invention;
Fig. 7 shows a schematic diagram of a bottom planar structure of the present invention;
Fig. 8 shows a schematic diagram of an overall operating system of the present invention;

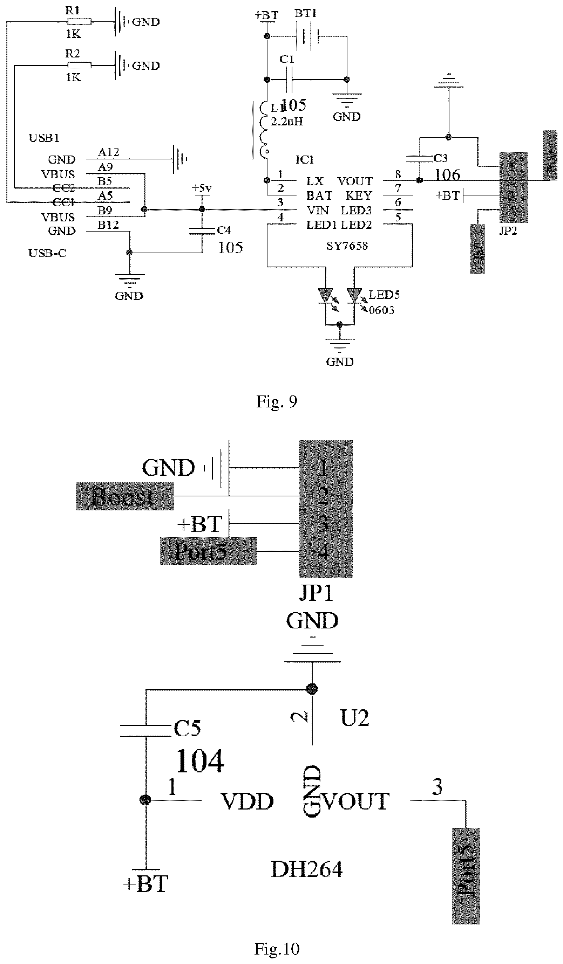

Fig. 9 shows a schematic diagram of an overall charging and boosting circuit of the present invention;
Fig. 10 shows a schematic diagram of the principle of a Hall detection circuit of the present invention;

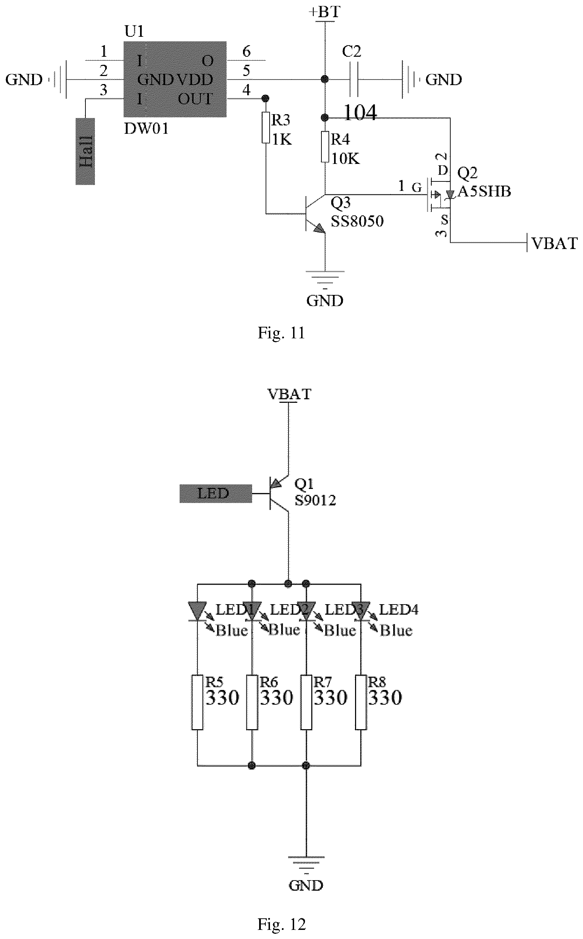

Fig. 11 shows a schematic diagram of the principle of a Hall switch circuit of the present invention;
Fig. 12 shows a schematic diagram of the principle of a circuit with music following a light of the present invention;

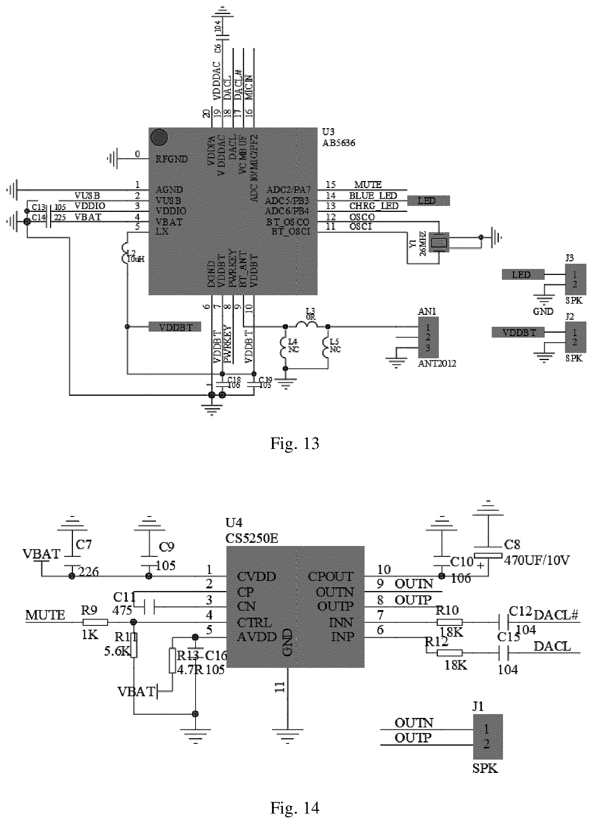

Fig. 13 shows a schematic diagram of the principle of a Bluetooth circuit of the present invention;
Fig. 14 shows a schematic diagram of the principle of an amplifying circuit of the present invention;

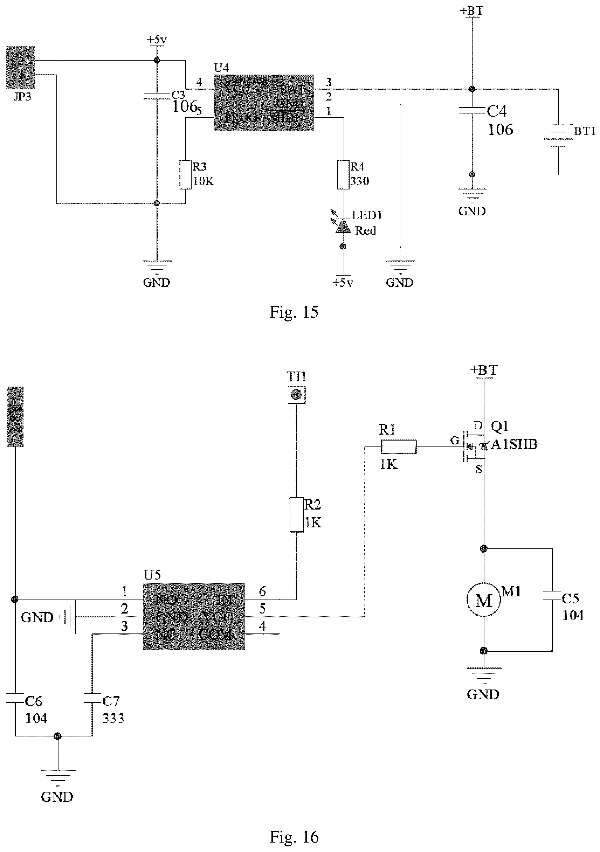

Fig. 15 shows a schematic diagram of the principle of a charging circuit of the present invention;
Fig. 16 shows a schematic diagram of the principle of a touch control circuit of the present invention;

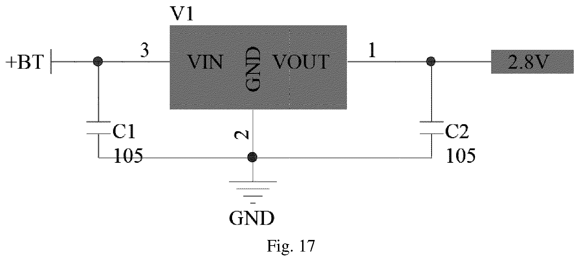

Fig. 17 shows a schematic diagram of the principle of a voltage stabilizing circuit of the present invention.

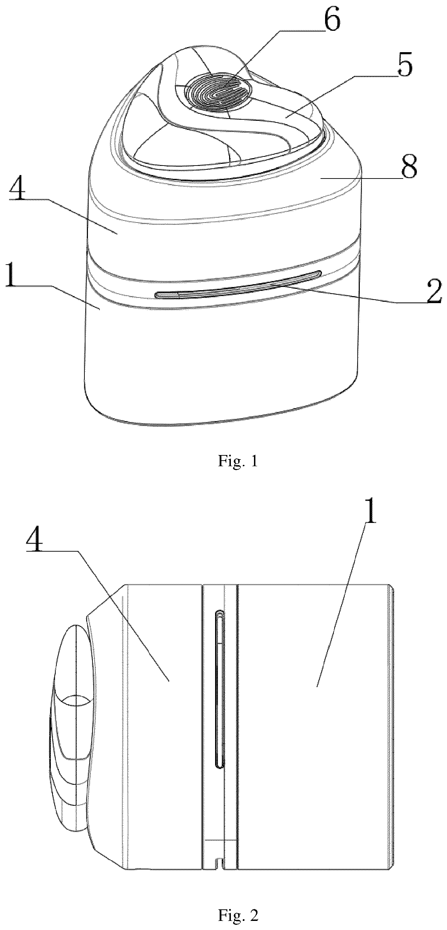

In the figures 1. device body; 2. sound outlet hole; 3. anti-slip pad; 4. upper cover; 5. vibrating massaging apparatus; 6. touch switch; 7. TYPE-C charging port; 8. LED light belt.

### DETAILED DESCRIPTION OF EMBODIMENTS

The following clearly and completely describes the technical solutions in embodiments of the present invention in conjunction with the drawings in the embodiments of the present invention. Obviously, the described embodiments are only a part of the embodiments of the present invention, rather than all embodiments. Based on the embodiments of the present invention, all other embodiments obtained by a person skilled in the art without inventive work shall fall within the scope of protection of the present invention.

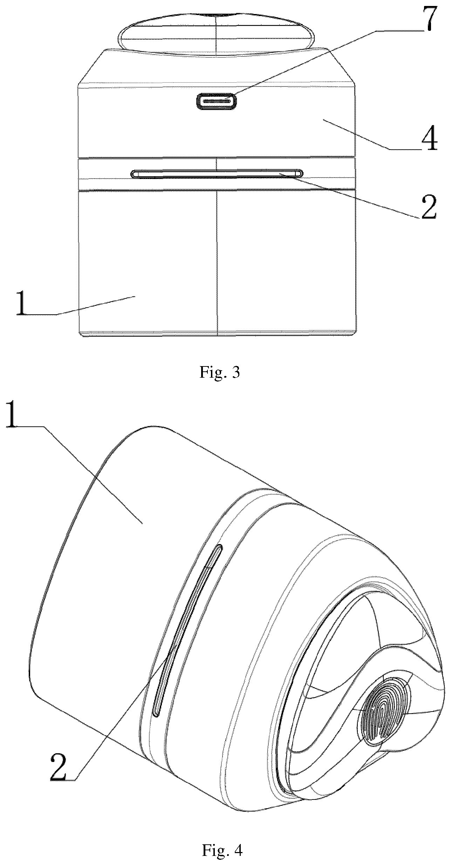

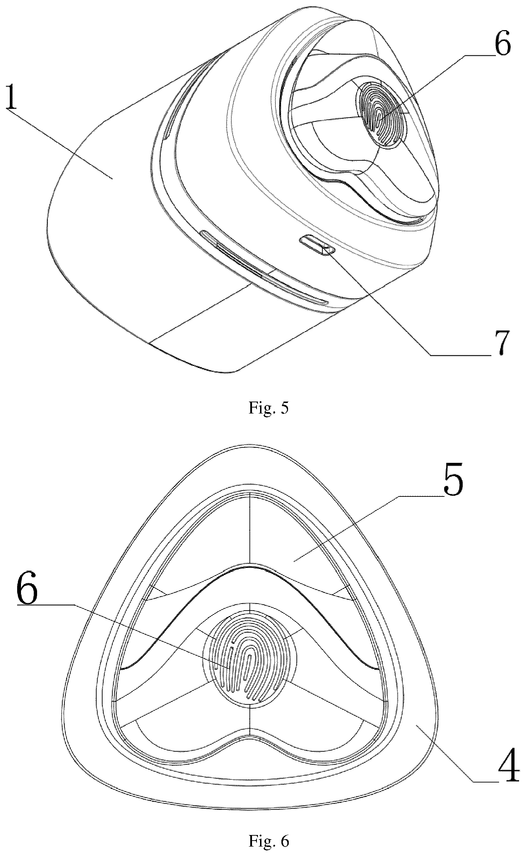

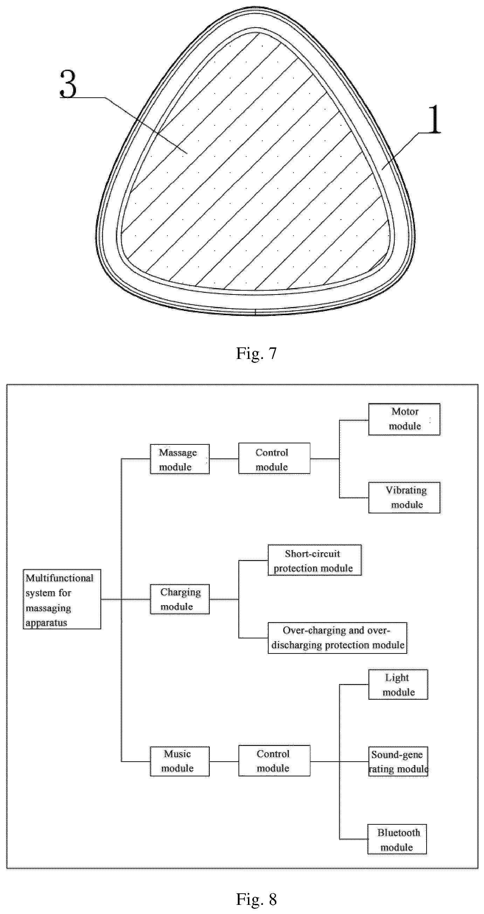

Please refer to Figs. 1-8, in this embodiment: a device for massaging a full face multifunctionally and musically includes a device body 1. The device body 1 can carry the installation of the remaining components. The device body 1 is provided with a sound outlet hole 2 at one side end face of the periphery. The sound outlet hole 2 may be used as a channel for an internal acoustic structure to propagate sound. The device body 1 is provided with an anti-slip pad 3 at one side end face of the bottom end. With the anti-slip pad 3, the device may be placed stable and anti-slip. The device body 1 is provided with an upper cover 4 at one side end face of the upper end. The upper cover 4 may separate the upper and lower structures of the device for different purposes. The upper cover 4 is provided with a vibrating massaging apparatus 5 at one side end face of the top. The vibrating massaging apparatus 5 may perform high-frequency vibration massage on a face. The vibrating massaging apparatus 5 is provided with a touch switch 6 at the end face of the center. The touch switch 6 may be used as a control structure for the device. The upper cover 4 is provided with a TYPE-C charging port 7 at one side end face. The TYPE-C charging port 7 is a charging structure of the device. The upper cover 4 is provided with an LED light belt 8. The LED light belt 8 can cooperate with music to create a cozy atmosphere, and also be used as an indicator of the operating state of the device.

It should be noted that a multifunctional system of the massaging apparatus of the device is specified as follows: the multifunctional system includes a massage module, a charging module, and a music module. The massage module includes a control module, a motor module, and a vibrating module. The charging module includes a short-circuit protection module and an over-charging and over-discharging protection module. The music module includes a control module, a light module, a sound-generating module, and a Bluetooth module. An output terminal of the motor module in the massage module is connected to the vibrating module. An output terminal of the short-circuit protection module in the charging protection module is connected to the over-charging and over-discharging protection module. An output terminal of the light module in the music module is connected to the sound-generating module. An output terminal of the control module is connected to the motor module and the Bluetooth module. The motor module provides a driving force for the vibrating module. The vibrating module directly contacts a user as a vibrating element. The short-circuit protection module may provide protection in the event of a short circuit in the device. The over-charging and over-discharging protection module may provide protection when the device is overcharged or anti-power. The light module may be used as the light-emitting element of the device. The sound-generating module may be configured to play music. The Bluetooth module may be connected to a cell phone. The control module may be used as a control element of the music module and the massage module.

In this embodiment, the device body 1 and a finger grip part of the upper cover 4 are made of a frosted matte ABS plastic so that the friction of the grip part is increased, thereby achieving the effect of anti-slip. The device body 1, the upper cover 4, and the vibrating massaging apparatus 5 as a whole adopt a heart-shaped triangular external structure with an upward tip. The vibrating massaging apparatus 5 is movably connected to the device body 1 through magnetism. A heart-shaped structure is arranged, which may further facilitate a user to hold the device. Three groups of sound outlet holes 2 are disclosed and distributed at each face of a triangle of the device body 1. The sound outlet holes 2 are fixedly connected to the device body 1. The three groups of the sound outlet holes 2 distributed at different faces are arranged so that the device has a better effect for sound outreach. The upper cover 4 is movably connected to the upper end of the device body 1 via a magnetic suction card so that it is convenient for the user to use an internal cream. The vibrating massaging apparatus 5 is fixedly connected to the inner part of the upper cover 4. The vibrating massaging apparatus 5 is fixedly mounted, so that the vibrating massaging apparatus 5 may be kept stable and fixed during vibrating operation. The anti-slip pad 3 is fixedly connected to the bottom end of the device body 1 by bonding. The anti-slip pad 3 is fixedly connected by bonding so that the anti-slip pad 3 may be kept to be stably and fixedly connected at the bottom end of the device body 1 during the vibrating operation.

The working principle and the use process of the present invention are as follows: the user first dials off the upper cover 4. The cream is evenly applied to a face skin. With the touch switch 6, the vibration massage apparatus 5 starts to make friction on the face skin. If the user needs to use a music function, the user turns on a cell phone for search and being connected to a Bluetooth KDE-5034. After linkage, the user can normally play the music. After the user removes the upper cover 4, the music starts to be played. The music pauses after the upper cover 4 is put back to the device body 1. Because the magnetic suction card, the vibrating massaging apparatus 5 automatically charged, and the device body 1 are arranged, when the device needs to be charged, it only needs to insert a TYPE-C charging cable into the TYPE-C charging port 7, and then put back to the upper end of the device body 1 the vibrating massaging apparatus 5 which is taken out, fix the vibrating massaging apparatus 5 through a magnetic suction and charge the vibrating massaging apparatus 5 automatically. When the vibrating massaging apparatus 5 is put back to the device body 1, magnetism is detected by Hall to trigger a circuit to be powered off. when the vibrating massaging apparatus 5 is taken away from the device body 1 for use, with the Hall detection switching principle, when the massaging apparatus is put back to the device body, the magnetism is detected by Hall to trigger the circuit to be powered off. When the massaging apparatus is taken away from the device body for use, magnetic disappearance is detected by Hall to trigger the circuit to be powered on and automatically play the music. During the device is charged, a red light of the LED light belt 8 is turned on. When the device is charged full, the red light is turned off. It should be noted that the device can not be washed, and is not suitable for use of children.

The invention is defined by the claims.

## Claims

1. A device for massaging a full face multifunctionally and musically, comprising a device body (1), wherein the device body (1) is provided with a sound outlet hole (2) at one side end face of a periphery, the device body (1) is provided with an anti-slip pad (3) at one side end face of a bottom end, the device body (1) is provided with an upper cover (4) at one side end face of an upper end, the upper cover (4) is provided with a vibrating massaging apparatus (5) at one side end face of a top, the vibrating massaging apparatus (5) is provided with a touch switch (6) at an end face of a center, the upper cover (4) is provided with a TYPE-C charging port (7) at one side end face, and the upper cover (4) is provided with an LED light belt (8) on an end face of an upper side of a periphery.

2. The device for massaging the full face multifunctionally and musically according to claim 1, wherein the device body (1) and a finger grip part of the upper cover (4) are made of a frosted matte ABS plastic.

3. The device for massaging the full face multifunctionally and musically according to claim 1, wherein the device body (1), the upper cover (4), and the vibrating massaging apparatus (5) as a whole adopt a heart-shaped triangular external structure with an upward tip, the vibrating massaging apparatus (5) is connected to the device body (1) via a magnetic suction, and the vibrating massaging apparatus (5) is movably connected to the device body (1).

4. The device for massaging the full face multifunctionally and musically according to claim 1, wherein three groups of sound outlet holes (2) are disclosed and distributed at each face of a triangle of the device body (1), respectively and the sound outlet holes are fixedly mounted and connected to the device body (1).

5. The device for massaging the full face multifunctionally and musically according to claim 1, wherein the upper cover (4) is movably connected to the upper end of the device body (1) by a magnetic suction card.

6. The device for massaging the full face multifunctionally and musically according to claim 1, wherein the vibrating massaging apparatus (5) is fixedly mounted and connected to an inner part of the upper cover (4).

7. The device for massaging the full face multifunctionally and musically according to claim 1, wherein the anti-slip pad (3) is fixedly connected to a bottom end of the device body (1) by bonding.

## Patentansprüche

1. Eine multifunktionelle und musikalische Massagevorrichtung für das ganze Gesicht, umfassend einen Gerätekörper (1), wobei an einer seitlichen Endfläche eines Umfangs des Gerätekörpers (1) eine Schallauslassöffnung (2) angeordnet ist, an einer seitlichen Endfläche eines unteren Endes des Gerätekörpers (1) ein Rutschsicherungspolster (3) angeordnet ist, an einer seitlichen Endfläche eines oberen Endes des Gerätekörpers (1) ein Oberteil (4) angeordnet ist, an einer seitlichen Endfläche einer Oberseite des Oberteils (4) eine Vibrationsmassagevorrichtung (5) angeordnet ist, an einer Endfläche einer Mitte der Vibrationsmassagevorrichtung (5) ein Berührungsschalter (6) angeordnet ist, an einer seitlichen Endfläche des Oberteils (4) ein TYPE-C-Ladeanschluss (7) angeordnet ist und an einer Endfläche einer oberen Seite eines Umfangs des Oberteils (4) ein LED-Leuchtstreifen (8) angeordnet ist.

2. Die multifunktionelle und musikalische Massagevorrichtung für das ganze Gesicht nach Anspruch 1, wobei der Gerätekörper (1) und ein Fingergriffteil des Oberteils (4) aus mattiertem ABS-Kunststoff gefertigt sind.

3. Die multifunktionelle und musikalische Massagevorrichtung für das ganze Gesicht nach Anspruch 1, wobei der Gerätekörper (1), das Oberteil (4) und die Vibrationsmassagevorrichtung (5) insgesamt eine herzförmige dreieckige Außenstruktur mit nach oben zeigender Spitze aufweisen, die Vibrationsmassagevorrichtung (5) über eine Magnetabsaugung mit dem Gerätekörper (1) verbunden ist und die Vibrationsmassagevorrichtung (5) beweglich mit dem Gerätekörper (1) verbunden ist.

4. Die multifunktionelle und musikalische Massagevorrichtung für das ganze Gesicht nach Anspruch 1, wobei drei Gruppen von Schallauslassöffnungen (2) offenbart sind und jeweils an jeder Fläche eines Dreiecks des Gerätekörpers (1) verteilt sind und die Schallauslassöffnungen fest an dem Gerätekörper (1) montiert und mit diesem verbunden sind.

5. Die Mmultifunktionelle und musikalische Massagevorrichtung für das ganze Gesicht nach Anspruch 1, wobei das Oberteil (4) beweglich über eine Magnetabsaugungsrastverbindung mit dem oberen Ende des Gerätekörpers (1) verbunden ist.

6. Die multifunktionelle und musikalische Massagevorrichtung für das ganze Gesicht nach Anspruch 1, wobei die Vibrationsmassagevorrichtung (5) fest an einem inneren Teil des Oberteils (4) montiert und mit diesem verbunden ist.

7. Die multifunktionelle und musikalische Massagevorrichtung für das ganze Gesicht nach Anspruch 1, wobei das Rutschsicherungspolster (3) fest durch Kleben mit einem unteren Ende des Gerätekörpers (1) verbunden ist.

## Revendications

1. Dispositif de massage de visage entier multifonctionnel et musical, comprenant un corps de dispositif (1), ledit corps de dispositif (1) étant pourvu d'un orifice de sortie sonore (2) sur une face latérale de sa périphérie, ledit corps de dispositif (1) étant pourvu d'un patin antidérapant (3) sur une face latérale de son extrémité inférieure, ledit corps de dispositif (1) étant pourvu d'un couvercle supérieur (4) sur une face latérale de son extrémité supérieure, ledit couvercle supérieur (4) étant pourvu d'un appareil de massage vibrant (5) sur une face supérieure, ledit appareil de massage vibrant (5) étant pourvu d'un interrupteur tactile (6) sur une face centrale, ledit couvercle supérieur (4) étant pourvu d'un port de charge TYPE-C (7) sur une face latérale, et ledit couvercle supérieur (4) étant pourvu d'une bande lumineuse LED (8) sur une face supérieure périphérique.

2. Le dispositif de massage de visage entier multifonctionnel et musical selon la revendication 1, dans lequel le corps de dispositif (1) et une partie de préhension digitale du couvercle supérieur (4) sont réalisés en plastique ABS mat sablé.

3. Le dispositif de massage de visage entier multifonctionnel et musical selon la revendication 1, dans lequel le corps de dispositif (1), le couvercle supérieur (4) et l'appareil de massage vibrant (5) adoptent dans leur ensemble une structure externe triangulaire en forme de coeur avec une pointe orientée vers le haut, l'appareil de massage vibrant (5) étant relié au corps de dispositif (1) par une aspiration magnétique et étant relié de manière mobile audit corps de dispositif (1).

4. Le dispositif de massage de visage entier multifonctionnel et musical selon la revendication 1, dans lequel trois groupes d'orifices de sortie sonore (2) sont disposés respectivement sur chaque face d'un triangle du corps de dispositif (1), lesdits orifices de sortie sonore étant fixés et connectés audit corps de dispositif (1).

5. Le dispositif de massage de visage entier multifonctionnel et musical selon la revendication 1, dans lequel le couvercle supérieur (4) est relié de manière mobile à l'extrémité supérieure du corps de dispositif (1) par un dispositif de verrouillage magnétique.

6. Le dispositif de massage de visage entier multifonctionnel et musical selon la revendication 1, dans lequel l'appareil de massage vibrant (5) est fixé et relié à une partie intérieure du couvercle supérieur (4).

7. Le dispositif de massage de visage entier multifonctionnel et musical selon la revendication 1, dans lequel le patin antidérapant (3) est fixé à l'extrémité inférieure du corps de dispositif (1) par collage.
